# EUROPEAN PATENT SPECIFICATION

(11) **EP 1 598 093 B1**
(45) Date of publication and mention of the grant of the patent: **02.11.2016**
(21) Application number: 05253091.2
(22) Date of filing: 19.05.2005
(51) Int. Cl.: A61N 1/362, A61N 1/365, A61N 1/39, A61B 5/00, A61B 5/053

(54) **System for automated fluid monitoring**
Automatisiertes System zur Überwachung von Flüssigkeiten
Système automatisé de surveillance de fluides

(30) Priority: 19.05.2004 US 850589
(43) Date of publication of application: 23.11.2005
(73) Proprietor: PACESETTER, INC., Sylmar, CA 91342-9221 (US)
(72) Inventor: Kroll, Mark W., Simi Valley, CA 93065 (US)
(74) Representative: Noble, Nicholas

(56) References cited:
- EP-A2- 1 291 036
- US-B2- 6 595 927

## Description

The invention relates generally to implantable cardiac devices, and particularly to systems for using such devices to measure body impedance parameters.

An implantable cardiac device is a medical device that is implanted in a patient to monitor electrical activity of a heart and to deliver appropriate electrical and/or drug therapy, as required. Implantable cardiac devices include, for example, pacemakers, cardioverters, defibrillators, and the like. The term "implantable cardioverter defibrillator" or simply "ICD" is used hereinafter to refer to any implantable cardiac device.

The electrical therapy produced by an ICD can include, for example, pacing pulses, cardioverting pulses, and/or defibrillator pulses. The ICD is used to both provide treatment for the patient and to inform the patient and medical personnel of the status of the patient and the treatment. Status information is often provided via a telemetry system that communicates with a close-by external system.

Body electrical impedance is well-established as a useful indicator of fluid balance in congestive heart failure (CHF) patients. Body electrical impedance is particularly useful as a diagnostic tool for serially monitoring individual patients. As fluid levels increase, decreased body impedance is observed. This increase in fluids and reduction of impedance is associated with impending CHF decompensation.

There have been investigations into the possibility of using certain types of ICDs to measure body electrical impedance. Studies undertaken in Hong Kong measured impedance between a defibrillation lead and a modified pacemaker. The results of such studies confirmed that reduced impedance correlates with worsening status of CHF patients. These studies used a low frequency (minute ventilation type) signal and required that a defibrillation lead be used.

U.S. Patent 6,473,640 to Erlebacher discloses an implantable device for monitoring of congestive heart failure. An electrical signal is generated to obtain a single or dual frequency measurement of venous and pulmonary impedance. Erlebacher uses an accelerometer to determine body position and then correlates and sorts the impedance measurements based on posture.

U.S. Patent 6,104,949, also to Pitts-Crick et al., shows a system that senses trans-thoracic impedance and uses a posture sensor to determine when the patient is lying down.

U.S. Patent 5,282,840 to Hudrlik discloses a multiple frequency impedance measuring system to detect ischemia. U.S. Patent 5,876,353 to Riff, U.S. Patents 5,957,861 and 6,512,949 to Combs et al., and U.S. Patent 6,595,927 and U.S. Published Application US2003/0023184 A1 of Pitts-Crick et al. disclose additional systems that measure impedance with an implantable device.

The inventor has determined that low frequency signals of the type used in the Hong Kong studies have limited accuracy in measuring body impedance, and are highly sensitive to posture variations.

Therefore, there is a need for a system for automatically and accurately monitoring body impedance and using the results as a diagnostic tool for evaluating patient health and treatment strategies.

US 6,595,927 details a method of diagnosing pulmonary congestion. At least one decrease in a trans-thoracic impedance value from a baseline trans-thoracic impedance value is sensed. At least one increase in a heart rate value from a baseline heart rate value is also sensed. Pulmonary congestion is diagnosed if the decrease in the trans-thoracic impedance value corresponding to the increase in the heart rate does not increase after a predetermined interval. Systems and programs incorporating the method are also provided.

EP1291036 describes how an implantable cardiac stimulation device is programmed to administer pacing therapy in response to a change in a patient's position and a drop in blood pressure. The stimulation device is equipped with a position sensor to sense a position parameter indicative

of when a patient changes from a supine position to an upright position and a pressure sensor to sense a pressure parameter indicative of a patient's blood pressure. The device administers cardiac pacing therapy to the patient based on both the position parameter and the pressure parameter.

According to the invention, there is provided a system for monitoring a patient using an implantable cardiac device (ICD), comprising: monitoring means for sensing aposition of the patient and making a determination of when the patient assumes a predetermined repose position for at least a predetermined period of time; control means for initiating a plurality of measurements of thoracic impedance after making the determination that the patient is in the predetermined repose position; impedance measuring means for obtaining the plurality of measurements of thoracic impedance by transmitting a signal on at least one ICD lead and sensing a received signal at a point separated from the ICD lead; and processing means for calculating, using the measurements, at least one quantity related to thoracic impedance.

Disclosed is a method of operating an implantable cardiac device (ICD) to monitor a patient, the method comprising: monitoring a position of the patient and making a determination of when the patient assumes a predetermined repose position for at least a predetermined period of time; initiating a plurality of measurements of thoracic impedance after making said determination that the patient is in said predetermined repose position; obtaining said plurality of measurements of thoracic impedance by transmitting a signal on at least one ICD lead and sensing a received signal at a point separated from said ICD lead; and using said measurements to calculate at least one quantity related to thoracic impedance.

What is described herein is an apparatus and method for improved monitoring of body impedance. In the disclosed exemplary arrangements, an implantable cardiac device and method monitors a patient's body impedance so as to diagnose medical problems, such as fluid balance problems in congestive heart failure patients. The device monitors the patient's position and determines an appropriate time for collecting body impedance data when the patient is in repose. The device then obtains a series of measurements of thoracic impedance by transmitting a signal on at least one lead, which may be a standard pacemaker lead, and sensing a signal received at a different point. The series of measurements are combined to obtain desired representations of thoracic impedance. In an embodiment, impedance is represented by an extracellular water (ECW) resistance value and an intracellular water (ICW) resistance value determined using a Cole calculation method.

A variety of high frequencies are used to measure impedance, thus increasing accuracy. Posture sensing optimizes the measurements and reduces power consumption during high frequency measurement.

Further features and advantages of the present invention as well as the structure and operation of various embodiments of the present invention are described in detail below with reference to the accompanying drawings.

The accompanying drawings, which are incorporated herein and form a part of the specification, illustrate the present invention and, together with the description, further serve to explain the principles of the invention and to enable a person skilled in the pertinent art to make and use the invention.
FIG. 1 is a diagram illustrating an ICD in electrical communication with at least three leads implanted into a patient's heart for optionally delivering stimulation and therapy, and for monitoring body impedance according to an embodiment of the present invention.
FIG. 2 is a block diagram of an ICD that incorporates the present invention and can optionally provide cardioversion, defibrillation and pacing stimulation as well as monitoring body impedance.
FIG. 3 shows an arrangement of ICD leads, including an atrial bipolar pair and a ventricular bipolar pair. In one embodiment of the invention, these leads are used as parallel impedance paths to the ICD can (i.e. ICD housing) for measuring body impedance.
FIG. 4 is a partial circuit diagram showing one embodiment of a circuit for measurement of body impedance using a lead arrangement of the type shown in Figure 3.
FIG. 5 shows a left ventricular lead arrangement. In an embodiment of the invention, these leads are used as impedance paths to the ICD can for measuring body impedance.
FIG. 6 is a partial circuit diagram showing one embodiment of a circuit for measurement of body impedance using a lead arrangement of the type shown in Figure 5.
FIG. 7 is a flow chart of a method according to one exemplary arrangement of the invention.
FIG. 8 is an electrical circuit model of cellular resistance useful in understanding operation of the invention.
FIGS. 9a and 9b are typical plots of real and imaginary components of impedance, respectively, for a test frequency of 5 to 500 kHz.
FIG. 10 is a typical Cole plot incorporating resistance and reactance information.

The present invention will now be described with reference to the accompanying drawings. In the drawings, like reference numbers may indicate identical or functionally similar elements. Additionally, the left-most digit(s) of a reference number may identify the drawing in which the reference number first appears.

It would be apparent to one of skill in the art that the present invention, as described below, may be implemented in many different embodiments of hardware, software, firmware, and/or the entities illustrated in the figures. Any actual software and/or hardware described herein is not limiting of the present invention. Thus, the operation and behavior of the present invention will be described with the understanding that modifications and variations of the embodiments are possible, given the level of detail presented herein.

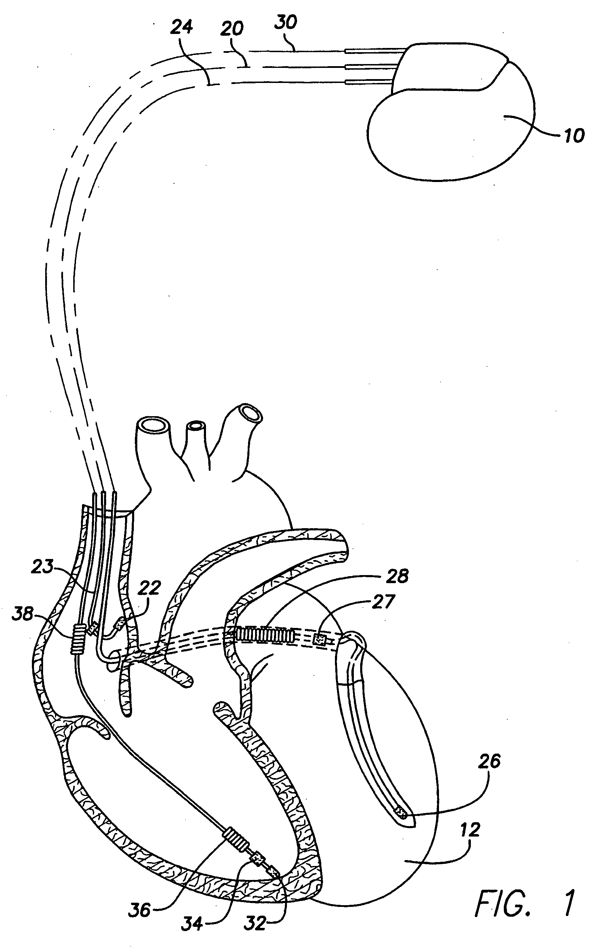

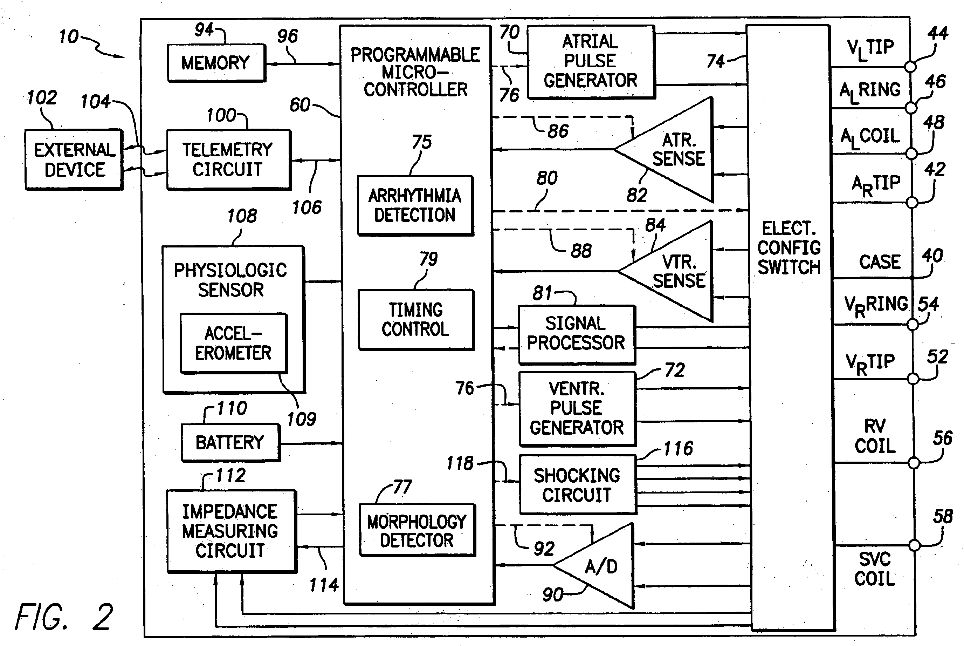

Before describing exemplary embodiments of the invention in detail, it is helpful to describe an example environment in which the invention may be implemented. The present invention is particularly useful in the environment of an implantable cardiac device. Implantable cardiac devices include, for example, pacemakers, cardioverters and defibrillators. The term "implantable cardiac device" or simply "ICD" is used herein to refer to any implantable cardiac device or implantable cardioverter defibrillator. FIGS. 1 and 2 illustrate such an environment.

As shown in FIG. 1, there is an exemplary ICD 10 in electrical communication with a patient's heart 12 by way of three leads, 20, 24 and 30, suitable for delivering multi-chamber stimulation and pacing therapy. To sense atrial cardiac signals and to provide right atrial chamber stimulation therapy, ICD 10 is coupled to Implantable right atrial lead 20 having an atrial tip electrode 22 and an atrial ring electrode 23, which are typically implanted in the patient's right atrial appendage.

To sense left atrial and ventricular cardiac signals and to provide left- chamber pacing therapy, ICD 10 is coupled to "coronary sinus" lead 24 designed for placement in the "coronary sinus region" via the coronary sinus for positioning a distal electrode adjacent to the left ventricle and/or additional electrode(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible by the coronary sinus.

Accordingly, exemplary coronary sinus lead 24 is designed to receive atrial and ventricular cardiac signals and to deliver left ventricular pacing therapy using at least a left ventricular tip electrode 26, left atrial pacing therapy using at least a left atrial ring electrode 27, and shocking therapy using at least a left atrial coil electrode 28.

ICD 10 is also shown in electrical communication with the patient's heart 12 by way of an implantable right ventricular lead 30 having, in this embodiment, a right ventricular tip electrode 32, a right ventricular ring electrode 34, a right ventricular (RV) coil electrode 36, and a superior vena cava (SVC) coil electrode 38. Typically, right ventricular lead 30 is transvenously inserted into heart 12 so as to place the right ventricular tip electrode 32 in the right ventricular apex so that RV coil electrode 36 will be positioned in the right ventricle and SVC coil electrode 38 will be positioned in the superior vena cava. Accordingly, right ventricular lead 30 is capable of receiving cardiac signals and delivering stimulation in the form of pacing and shock therapy to the right ventricle.

Figure 2 shows a simplified block diagram of ICD 10, which is capable of treating both fast and slow arrhythmias with stimulation therapy, including cardioversion, defibrillation, and pacing stimulation. While a particular multi-chamber device is shown, it is shown for illustration purposes only, and one of skill in the art could readily duplicate, eliminate or disable the appropriate circuitry in any desired combination to provide a device capable of treating the appropriate chamber(s) with the desired cardioversion, defibrillation and pacing stimulation.

A housing 40 of ICD 10, shown schematically in FIG. 2, is often referred to as the "can," "case" or "case electrode" and may be programmably selected to act as the return electrode for all "unipolar" modes. Housing 40 may further be used as a return electrode alone or in combination with one or more of coil electrodes, 28, 36, and 38 for shocking purposes. Housing 40 further includes a connector (not shown) having a plurality of terminals, 42, 44, 46, 48, 52, 54, 56, and 58 (shown schematically and, for convenience, the names of the electrodes to which they are connected are shown next to the terminals). As such, to achieve right atrial sensing and pacing, the connector includes at least a right atrial tip terminal (AR TIP) 42 adapted for connection to atrial tip electrode 22.

To achieve left chamber sensing, pacing and shocking, the connector includes at least a left ventricular tip terminal (VL TIP) 44, a left atrial ring terminal (AL RING) 46, and a left atrial shocking terminal (AL COIL) 48, which are adapted for connection to left ventricular ring electrode 26, left atrial tip electrode 27, and left atrial coil electrode 28, respectively.

To support right chamber sensing, pacing, and shocking the connector also includes a right ventricular tip terminal (VR TIP) 52, a right ventricular ring terminal (VR RING) 54, a right ventricular shocking terminal (RV COIL) 56, and an SVC shocking terminal (SVC COIL) 58, which are configured for connection to right ventricular tip electrode 32, right ventricular ring electrode 34, RV coil electrode 36, and SVC coil electrode 38, respectively.

At the core of ICD 10 is a programmable microcontroller 60 that controls the various modes of stimulation therapy. Microcontroller 60 typically includes a microprocessor, or equivalent control circuitry, designed specifically for controlling the delivery of stimulation therapy and can further include RAM or ROM memory, logic and timing circuitry, state machine circuitry, and I/O circuitry. Typically, microcontroller 60 includes the ability to process or monitor input signals (data) as controlled by a program code stored in a designated block of memory. The details of the design of microcontroller 60 are not critical to the present invention. Rather, any suitable microcontroller 60 can be used to carry out the functions described herein. In specific embodiment of the present invention, microcontroller 60 performs some or all of the steps associated with tracking battery usage in accordance with the present invention.

Representative types of control circuitry that may be used with the invention include the microprocessor-based control system of U.S. Patent No. 4,940,052 (Mann et al.) and the state-machines of U.S. Patent Nos. 4,712,555 (Thomander et al.) and 4,944,298 (Sholder). For a more detailed description of the various timing intervals used within the ICD's and their inter-relationship, see U.S. Patent 4,788,980 (Mann et al.).

As shown in Figure 2, an atrial pulse generator 70 and a ventricular pulse generator 72 generate pacing stimulation pulses for delivery by right atrial lead 20, right ventricular lead 30, and/or coronary sinus lead 24 via an electrode configuration switch 74. It is understood that in order to provide stimulation therapy in each of the four chambers of the heart, atrial and ventricular pulse generators 70 and 72, may include dedicated, independent pulse generators, multiplexed pulse generators, or shared pulse generators. Pulse generators 70 and 72 are controlled by microcontroller 60 via appropriate control signals 76 and 78, respectively, to trigger or inhibit the stimulation pulses.

Microcontroller 60 further includes timing control circuitry 79 which is used to control pacing parameters (e.g., the timing of stimulation pulses) as well as to keep track of the timing of refractory periods, PVARP intervals, noise detection windows, evoked response windows, alert intervals, and marker and channel timing. Examples of pacing parameters include, but are not limited to, atrio-ventricular (AV) delay, interventricular (RV-LV) delay, atrial interconduction (A-A) delay, ventricular interconduction (V-V) delay, and pacing rate.

Switch 74 includes a plurality of switches for connecting the desired electrodes to the appropriate I/O circuits, thereby providing complete electrode programmability. Accordingly, switch 74, in response to a control signal 80 from microcontroller 60, determines the polarity of the stimulation pulses (e.g., unipolar, bipolar, combipolar, etc.) by selectively closing the appropriate combination of switches (not shown) as is known in the art.

Atrial sensing circuits 82 and ventricular sensing circuits 84 may also be selectively coupled to right atrial lead 20, coronary sinus lead 24, and right ventricular lead 30, through switch 74 for detecting the presence of cardiac activity in each of the four chambers of the heart. Accordingly, the atrial (ATR. SENSE) and ventricular (VTR. SENSE) sensing circuits 82 and 84 may include dedicated sense amplifiers, multiplexed amplifiers, or shared amplifiers. Switch 74 determines the "sensing polarity" of the cardiac signal by selectively closing the appropriate switches, as is also known in the art. In this way, the clinician may program the sensing polarity independent of the stimulation polarity.

Each sensing circuit, 82 and 84, preferably employs one or more low power, precision amplifiers with programmable gain and/or automatic gairi control, band pass filtering, and a threshold detection circuit, as known in the art, to selectively sense the cardiac signal of interest. The automatic gain control enables ICD 10 to deal effectively with the difficult problem of sensing the low amplitude signal characteristics of atrial or ventricular fibrillation. Such sensing circuits, 82 and 84, can be used to determine cardiac performance values used in the present invention.

The outputs of atrial and ventricular sensing circuits 82 and 84 are connected to microcontroller 60 which, in turn, are able to trigger or inhibit atrial and ventricular pulse generators, 70 and 72, respectively, in a demand fashion in response to the absence or presence of cardiac activity, in the appropriate chambers of the heart. Sensing circuits 82 and 84, in turn, receive control signals over signal lines 86 and 88 from microcontroller 60 for purposes of measuring cardiac performance at appropriate times, and for controlling the gain, threshold, polarization charge removal circuitry (not shown), and timing of any blocking circuitry (not shown) coupled to the inputs of sensing circuits 82 and 84.

For arrhythmia detection, ICD 10 utilizes the atrial and ventricular sensing circuits 82 and 84 to sense cardiac signals to determine whether a rhythm is physiologic or pathologic. The timing intervals between sensed events (e.g., P-waves, R-waves, and depolarization signals associated with fibrillation which are sometimes referred to as "F-waves" or "Fib-waves") are then classified by microcontroller 60 by comparing them to a predefined rate zone limit (i.e., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy").

Microcontroller 60 utilizes arrhythmia detection circuitry 75 and morphology detection circuitry 77 to recognize and classify arrhythmia so that appropriate therapy can be delivered. In the case where ICD 10 is intended to operate as a cardioverter, pacer or defibrillator, ICD 10 detects the occurrence of an arrhythmia and automatically applies an appropriate electrical therapy to the heart aimed at terminating the detected arrhythmia.

ICD 10 preferably incorporates signal processor 81, which includes a signal generator that generates a sensing signal to be transmitted to one or more of the leads shown in Figure 1 for body impedance measurement, and an impedance detector that receives the return impedance measurement signal from the leads, in a manner that will be described in more detail below. Signal processor 81 is advantageously coupled to switch 74 so that any desired electrode or electrodes may be coupled to the signal generator and impedance detector in signal processor 81. Signal processor 81 is also coupled to microcontroller 60 through an appropriate interface, so that microcontroller 60 can control signal processor 81 and receive impedance data from signal processor 81.

Shocking circuit 116 generates shocking pulses of low (up to 0.5 joules), moderate (0.5 - 10 joules), or high energy (11 to 40 joules), as controlled by microcontroller 60. Such shocking pulses are applied to the patient's heart 12 through at least two shocking electrodes (e.g., selected from left atrial coil electrode 28, RV coil electrode 36, and SVC coil electrode 38). As noted above, housing 40 may act as an active electrode in combination with RV electrode 36, or as part of a split electrical vector using SVC coil electrode 38 or left atrial coil electrode 28 (i.e., using the RV electrode as a common electrode).

Cardioversion shocks are generally considered to be of low to moderate energy level (so as to minimize pain felt by the patient), and/or synchronized with an R-wave and/or pertaining to the treatment of tachycardia. Defibrillation shocks are generally of moderate to high energy level (i.e., corresponding to thresholds in the range of 5-40 joules), delivered asynchronously (since R-waves may be too disorganized to be recognized), and pertaining exclusively to the treatment of fibrillation. Accordingly, microcontroller 60 is capable of controlling the synchronous or asynchronous delivery of the shocking pulses.

Cardiac signals are also applied to the inputs of an analog-to-digital (A/D) data acquisition system 90. Data acquisition system 90 is configured to acquire intracardiac electrogram signals, convert the raw analog data into a digital signal, and store the digital signals for later processing and/or telemetric transmission to an external device 102. Data acquisition system 90 is coupled to right atrial lead 20, coronary sinus lead 24, and right ventricular lead 30 through switch 74 to sample cardiac signals across any pair of desired electrodes.

Advantageously, data acquisition system 90 can be coupled to microcontroller 60, or other detection circuitry, for detecting an evoked response from heart 12 in response to an applied stimulus, thereby aiding in the detection of "capture." Capture occurs when an electrical stimulus applied to the heart is of sufficient energy to depolarize the cardiac tissue, thereby causing the heart muscle to contract. Microcontroller 60 detects a depolarization signal during a window following a stimulation pulse, the presence of which indicates that capture has occurred. Microcontroller 60 enables capture detection by triggering ventricular pulse generator 72 to generate a stimulation pulse, starting a capture detection window using timing control circuitry 79 within microcontroller 60, and enabling data acquisition system 90 via control signal 92 to sample the cardiac signal that falls in the capture detection window and, based on the amplitude, determines if capture has occurred.

The implementation of capture detection circuitry and algorithms are well known. See for example, U.S. Patent No. 4,729,376 (Decote, Jr.); U.S. Patent No. 4,708,142 (Decote, Jr.); U.S. Patent No. 4,686,988 (Sholder); U.S. Patent No. 4,969,467 (Callaghan et al.); and U.S. Patent No. 5,350,410 (Kleks et al.). The type of capture detection system used is not critical to the present invention.

Microcontroller 60 is further coupled to a memory 94 by a suitable data/address bus 96, wherein the programmable operating parameters used by microcontroller 60 are stored and modified, as required, in order to customize the operation of ICD 10 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart 12 within each respective tier of therapy. These operating parameters also include a value defining the number of escape cycles to wait after completion of a therapy before activating arrhythmia detection circuitry 75 to determine whether that therapy has arrested the arrhythmia.

Advantageously, the operating parameters of ICD 10 may be non-invasively programmed into memory 94 through a telemetry circuit 100 in telemetric communication with external device 102, such as a programmer, transtelephonic transceiver, or a diagnostic system analyzer. Telemetry circuit 100 is activated by microcontroller 60 by a control signal 106. Telemetry circuit 100 advantageously allows intracardiac electrograms and status information relating to the operation of ICD 10 (as contained in microcontroller 60 or memory 94) to be sent to external device 102 through an established communication link 104.

For examples of such devices, see U.S. Patent No. 4,809,697, entitled "Interactive Programming and Diagnostic System for use with Implantable Pacemaker" (Causey, III et al.); U.S. Patent No. 4,944,299, entitled "High Speed Digital Telemetry System for Implantable Device" (Silvian); and U.S. Patent No. 6,275,734, entitled "Efficient Generation of Sensing Signals in an Implantable Medical Device such as a Pacemaker or ICD" (McClure et al.).

In the preferred embodiment, ICD 10 further includes a physiologic sensor 108 that can be used to detect changes in cardiac performance or changes in the physiological condition of the heart. Accordingly, microcontroller 60 can respond by adjusting the various pacing parameters (such as rate, AV Delay, RV-LV Delay, V-V Delay, etc.) in accordance with the embodiments of the present invention. Microcontroller 60 controls adjustments of pacing parameters by, for example, controlling the stimulation pulses generated by the atrial and ventricular pulse generators 70 and 72. While shown as being included within ICD 10, physiologic sensor 108 may also be external to ICD 10, yet still be implanted within or carried by the patient. More specifically, sensor 108 can be located inside ICD 10, on the surface of ICD 10, in a header of ICD 10, or on a lead (which can be placed inside or outside the bloodstream).

Physiologic sensor 108 preferably includes one or more devices for determining body position. These may include one or more accelerometers, other types of micro-electromechanical systems (MEMS), and other known position sensing devices. In the embodiment shown, physiologic sensor 108 includes one or more accelerometers, identified as 3-D accelerometers 109, and may also include a magnetic field sensor associated with accelerometers 109. Accelerometer 109 may be used to detect position and movement of the patient, and may be used in a known manner to detect whether the patient is in repose, and preferably in particular whether the patient is supine. Physiologic sensor 108 may use accelerometer 109 to determine when the patient is supine, for example, according to the methods and apparatus disclosed in U.S. Patents 6,658,292, 6,625,493, and/or 6,466,821. It will be understood that the accelerometer(s) 109 represent a means for detecting whether a patient is in repose, and that other means and methods of detecting whether the patient is in repose may be implemented. What is important is not the principle of operation of physiologic sensor 108, but that it provides an indication of the patient's repose, e.g. whether the patient is supine.

ICD 10 additionally includes a battery 110 that provides operating power to all of the circuits shown in FIG. 2. For ICD 10, which employs shocking therapy, battery 110 must be capable of operating at low current drains for long periods of time, and then be capable of providing high-current pulses (for capacitor charging) when the patient requires a shock pulse. Battery 110 must also have a predictable discharge characteristic so that elective replacement time can be detected. Accordingly, ICD 10 preferably employs lithium/silver vanadium oxide batteries, as is true for most (if not all) current devices, although other battery technologies may be used.

ICD 10 may also include a magnet detection circuitry (not shown), coupled to microcontroller 60. It is the purpose of the magnet detection circuitry to detect when a magnet is placed over ICD 10, which magnet may be used by a clinician to perform various test functions of ICD 10 and/or to signal microcontroller 60 that the external programmer 102 is in place to receive or transmit data to microcontroller 60 through telemetry circuit 100.

As further shown in FIG.2, ICD 10 may include an impedance measuring circuit 112 which is enabled by microcontroller 60 via a control signal 114. The uses of the impedance measuring circuit 112 may include, but are not limited to, lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves, etc. The impedance measuring circuit 112 is advantageously coupled to switch 74 so that any desired electrode may be used. In some embodiments, impedance measuring circuit 112 may be advantageously combined with signal processor 81, which incorporates a specialized circuit adapted to support impedance measurement.

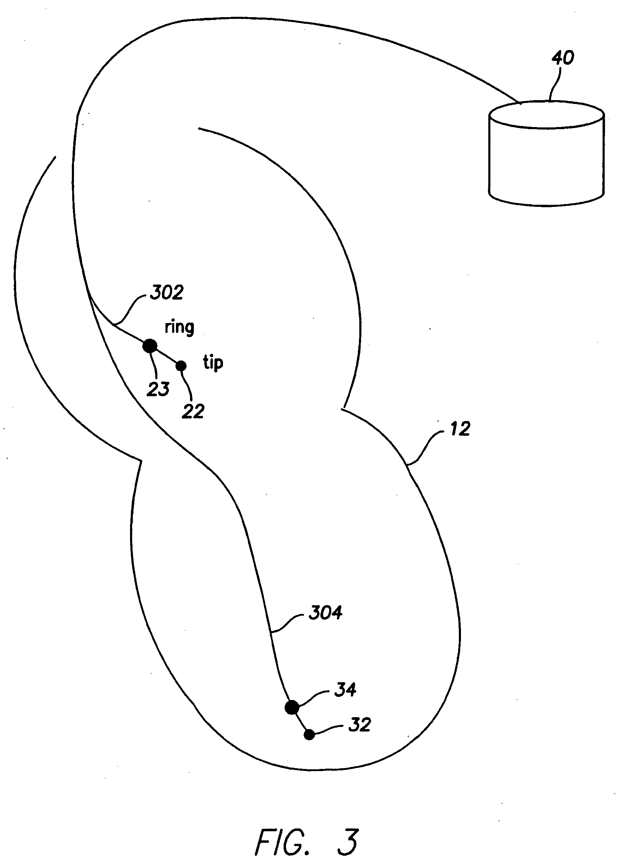

Figure 3 shows an arrangement of ICD leads including a right atrial bipolar pair 302 and a right ventricular bipolar pair 304 coupled to a heart 12. Right atrial bipolar pair 302 comprises atrial tip electrode 22 and atrial ring electrode 23. Right ventricular bipolar pair 304 comprises right ventricular tip electrode 32 and right ventricular ring electrode 34. In a first embodiment of the invention, these leads are used as parallel impedance paths to the housing or "can" 40 of an ICD for measuring body impedance.

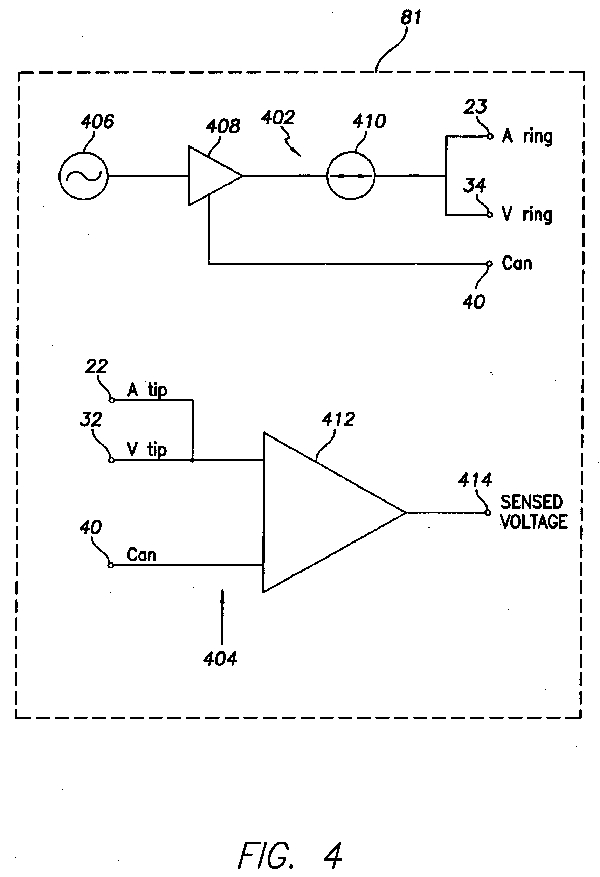

Figure 4 shows a more detailed view of part of the circuit in an embodiment of signal processor 81 (shown in Figure 2). In the embodiment of Figure 4, signal processor 81 comprises signal generator circuit 402 and sensing circuit 404. Signal generator circuit 402 and sensing circuit 404 are adapted for connection to the housing 40 or "can" (shown in Figures 2 and 3) for measurement of body impedance using a lead arrangement of the type shown generally in Figure 3, or a functionally equivalent lead arrangement.

In signal generator circuit 402, an AC signal source 406 generates an AC signal and transmits the signal to amplifier 408 which drives controlled current source 410 to produce a controlled current signal at the atrial ring electrode 23 and right ventricular ring electrode 34, which are connected in parallel to the output of controlled current source 410. These two electrodes are driven with reference to housing 40, thus producing a current between the housing 40 and the heart.

Sensing circuit 404 comprises amplifier 412 that produces a sensed voltage signal 414. Amplifier 412 has two inputs, a first input to which atrial tip electrode 22 and ventricular tip electrode 32 are connected in parallel, and a second reference input to which housing 40 is connected. The ratio of the sensed voltage 414 to the driven current produced by current source 410 is the impedance.

The ring electrodes 23 and 34 have a large surface area, and the use of these electrodes in parallel effectively simulates the use of the defibrillation coil. Further, the use of separate current driving electrodes and voltage sensing electrodes in this embodiment alleviates problems that would otherwise be experienced when using smaller electrodes.

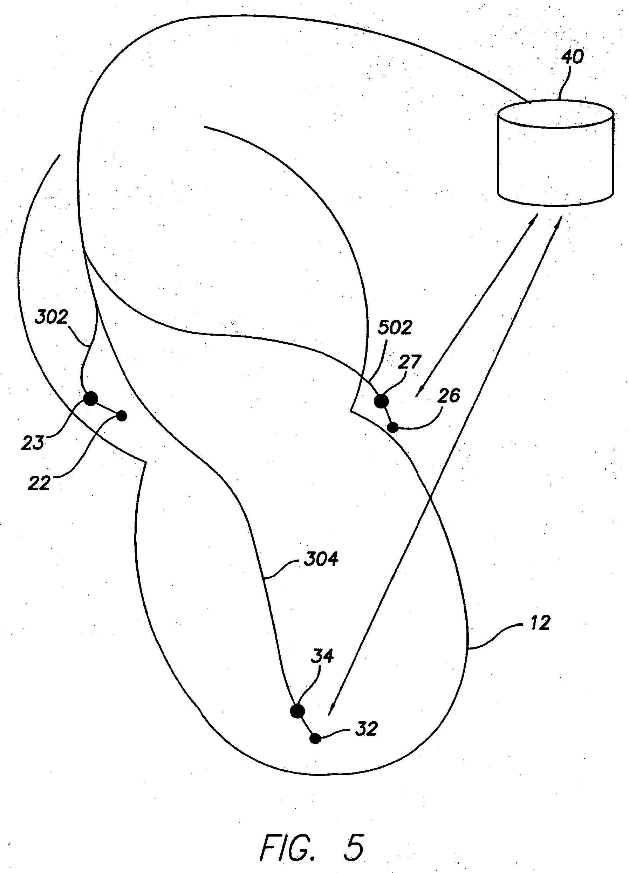

Figure 5 illustrates an alternative lead arrangement where left and right ventricular leads are used, in contrast to the use of right ventricular and atrial leads in the embodiment of Figure 3. In the embodiment of Figure 5, a left ventricular bipolar pair 502 comprises left ventricular tip electrode 26 and left ventricular ring electrode 27. To facilitate more accurate measurements of body impedance, the left ventricular tip electrode 26 and left ventricular ring electrode 27 are preferably placed closer together, as shown in Figure 5, than they might otherwise be in a system that is not intended to perform body impedance monitoring functions.

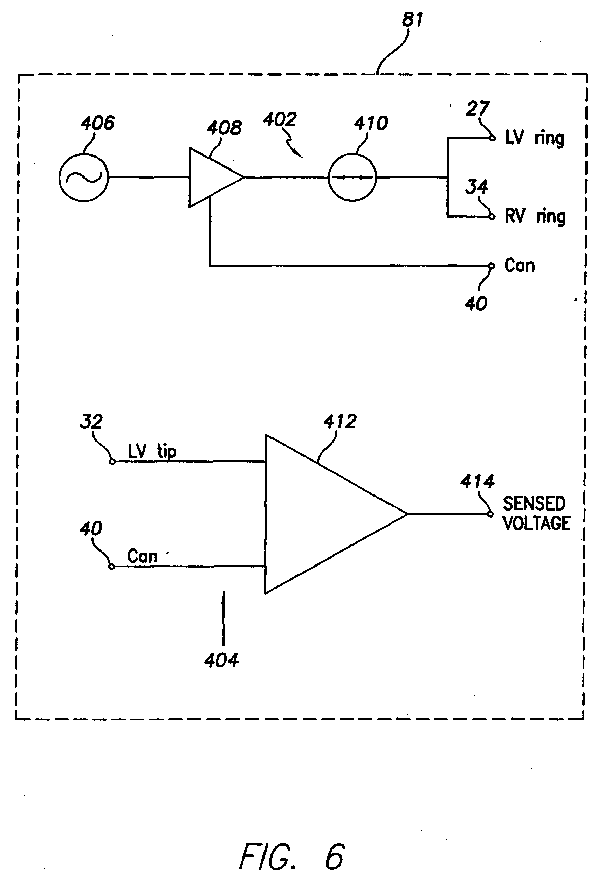

Figure 6 illustrates the circuit connections for signal processor 81 (shown in Figure 2) to operate with the lead arrangement of Figure 5. In the embodiment of Figure 6, signal processor 81 comprises signal generator circuit 402 and sensing circuit 404. Signal generator circuit 402 and sensing circuit 404 are adapted for connection to the housing 40 or "can" (shown in Figures 2 and 3) for measurement of body impedance using a lead arrangement of the type shown generally in Figure 5, or another functionally equivalent lead arrangement.

As described previously with reference to Figure 4, in signal generator circuit 402, an AC signal source 406 generates an AC signal and transmits the signal to amplifier 408 which drives controlled current source 410 to produce a controlled current signal. In the embodiment of Figure 6, the output of controlled current source 410 is connected in parallel to left ventricular ring electrode 27 and right ventricular ring electrode 34. These electrodes are driven in parallel with reference to housing 40, thus producing a current between the housing 40 and the heart.

As shown in Figure 6, sensing circuit 404 comprises amplifier 412 that produces a sensed voltage signal 414. Amplifier 412 has two inputs, a first input to which the left ventricular tip electrode 26 is connected, and a second reference input to which housing 40 is connected. The ratio of the sensed voltage 414 to the driven current produced by current source 410 is the impedance. As in the case of the Figure 4 embodiment, the ring electrodes 27 and 34 have a large surface area, and the use of these electrodes in parallel effectively simulates the use of the defibrillation coil. The use of separate current driving electrodes and voltage sensing electrodes also alleviates problems that would otherwise be experienced when using smaller electrodes. Another particular advantage of this embodiment is that the use for sensing of the left ventricular tip lead 26 alone, as opposed to a combination of leads as in Figure 4, will tend to sense the signal as it passes through more lung tissue and less cardiac tissue. In this way this embodiment provides a more focused measurement of lung fluid and thereby assists in the detection of the onset of pulmonary edema.

In the embodiments of Figure 3 and Figure 5, the system uses existing pacemaker leads for both transmitting and receiving the signals used to measure body impedance. The use of standard pacemaker leads is preferred, but the invention is not limited to embodiments using standard pacemaker leads. Specific additional leads could also be provided for this purpose, or leads used for purposes other than pacemaker operation could be used for this purpose.

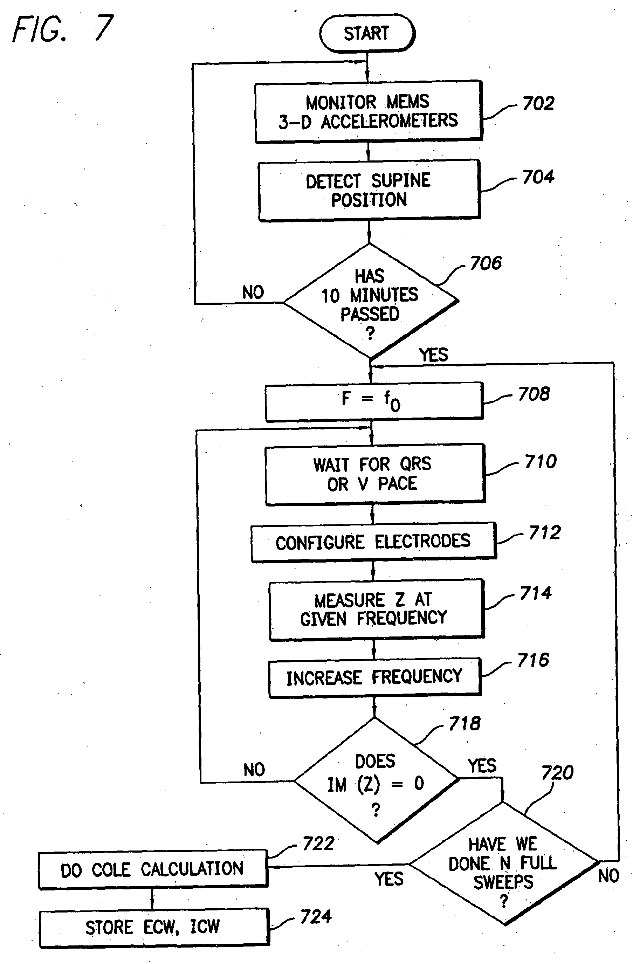

Figure 7 is a flow chart illustrating a process implemented in an exemplary embodiment of the invention. As shown in Figure 7, process 700 begins with step 702, where the physiologic sensor 108 and its 3-D accelerometers 109 (both shown in Figure 2) or their equivalent, such as other types of micro-electromechanical systems (MEMS) are monitored. In step 704, the system determines whether the patient is in repose, for example, supine. While various positions of repose could be used for measurement, preferably the system determines whether the patient is in a supine position, as the inventor has found that the most repeatable and accurate body impedance measurements are obtained when the patient has been in a supine position for several minutes and body fluids have stabilized.

In step 706, the system determines whether the patient has been in repose continuously for ten minutes. If not, control passes to step 702 and the system continues to monitor patient position. When the patient has been in the desired position for a selected time frame period. For example, in one embodiment the time period is set at ten minutes. The process continues at step 708 where the system begins an impedance measurement sweep by setting the impedance measurement frequency F to initial frequency f₀. The initial frequency may be set, for example, to 5 kHz.

In step 710 the system determines the timing of the next heartbeat by monitoring QRS, the electrical signal generated by a normal heartbeat, or Vpace, the pacing signal generated by the pacemaker. The system will use the leads for impedance measurement only during those times that the leads are not needed for heartbeat detection, pacing, cardioversion, or other operations. This selective operation and the timing control provided by the monitoring of QRS and Vpace makes it possible to use the existing pacemaker leads for this additional purpose without interfering with basic device operation.

Following the heartbeat, in step 712 the electrodes are configured for use in impedance measurement. This step preferably includes activating signal processor 81 (shown in Figure 2) and connecting the electrodes to the circuits incorporated therein using switch 74 (also shown in Figure 2).

In step 714, the impedance Z is measured at frequency F. In step 716, frequency F is increased by a predetermined increment, such as 1 kHz. In another embodiment, the predetermined increment may be a percentage of the range between the initial frequency and the expected ending frequency, such as one percent. Next, in step 718, the system determines whether the imaginary part of the impedance sensed in response to the current excitation frequency F is equal to or approximately equal to zero. When the imaginary part of the impedance reaches approximately zero, collection of additional data at higher frequencies would not be useful and the sweep is considered complete. The process then continues at step 720. If not, control passes to step 710 and steps 710 through 716 are repeated to obtain the next Z measurement in the sweep. In the impedance measuring process, preferably sensed voltage data is collected for each frequency F so that this data can be combined and processed at the conclusion of the sensing sweeps to accurately calculate Z and/or other useful quantities related to body impedance.

In step 720, the system determines whether N full sweeps have been completed, where N is a number of sweeps to be performed. For example, in an embodiment N may be equal to 10, so that the system collects ten sweeps before processing the collected data. A different number N may be selected if desired. The example uses N=10 sweeps in one embodiment because the inventor has determined that ten sweeps will generally provide sufficient data to permit averaging of the results, thus compensating for impedance variation resulting from breathing and other physiological processes occurring during the measurement cycle.

If the desired number of sweeps have not been completed, control passes to step 708, frequency F is re-initialized to f₀, for example to 5 kHz, and steps 710 through 718 are repeated to complete another sweep in the same manner described above. If N sweeps have been completed, the process continues with step 722 where a Cole calculation is performed in a manner that will be described in more detail below. The Cole calculation facilitates decompensation monitoring.

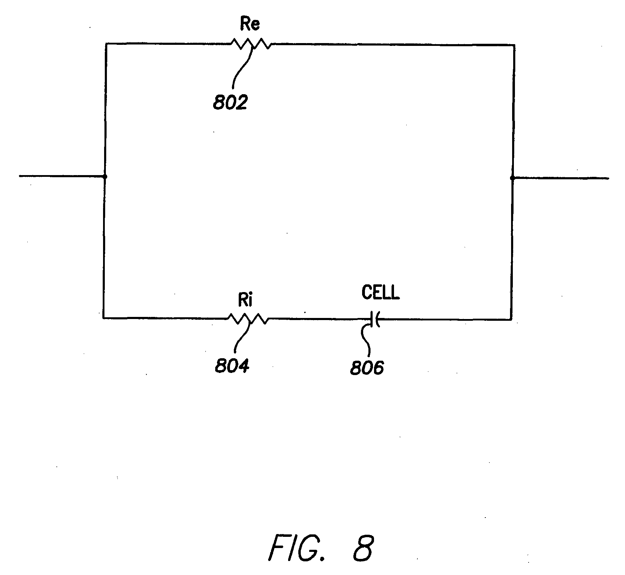

Finally, in step 724, values for extra-cellular water (ECW) resistance and intra-cellular water (ICW) resistance are stored for review and diagnostic use. These values may be calculated as described below with reference to Figure 8. The collected information regarding impedance quantities and body fluids is stored in memory 94 (see FIG. 2) and may be retrieved from storage using telemetry. Diagnosis based on the data may then be performed by an external system or through manual review of the data. In one embodiment, the system also monitors impedance levels and/or trends in impedance internally, and provides a warning indication to the patient or physician or performs a treatment function when an undesirable level or trend is observed.

Figure 8 is a model of cellular resistance useful in understanding the operation of the invention and the calculations performed. Extracellular fluid is modeled as a simple resistance 802, also designated Rₑ. Intracellular fluid is similarly modeled as resistance 804, also designated Rᵢ. The cell membrane has both capacitive and insulative properties, making tissue impedance frequency-dependent. Therefore, the model includes capacitance 806. In this simple model, capacitance 806 is shown in series with Rᵢ, and Re is parallel to the other two elements.

The value of ECW is proportional to the resistance (Ro) at f=0, which is equal to Rₑ. The resistance (R_{∞}) at f = ∞ is equal to the parallel combination of Rᵢ and Rₑ. By performing a standard electrical engineering calculation using the collected data, the value of Rᵢ can be calculated, thus giving the ICW estimate. The estimates of ECW and ICW provide a useful, simple indication of the level of body impedance that can be monitored over time to identify undesirable trends.

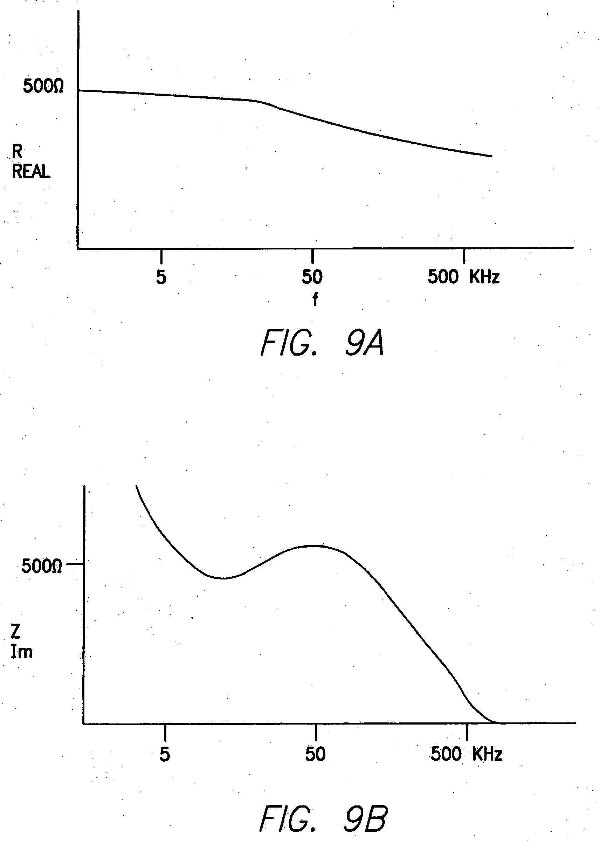

Figures 9A and 9B are typical plots of the real and imaginary components of impedance for a test frequency sweep of 5 kHz to 500 kHz. As can be seen in Figure 9A, the real component of the impedance drops slightly as the frequency increases. The imaginary component of the impedance, shown in Figure 9B, begins with an infinite reactance at low frequencies. As the frequency increases, it goes to a first local minimum at approximately 5 kHz. It then rises to a local maximum and then asymptotically approaches zero. The inventor has determined that the imaginary component drops near 5 Khz because the cells are able to conduct higher frequencies through their capacitive membranes which otherwise insulate at low frequencies. The imaginary impedance goes to zero at high frequencies because there are essentially no inductive components in the body, resulting in a purely capacitive and resistive response that produces a zero imaginary component at the higher frequencies.

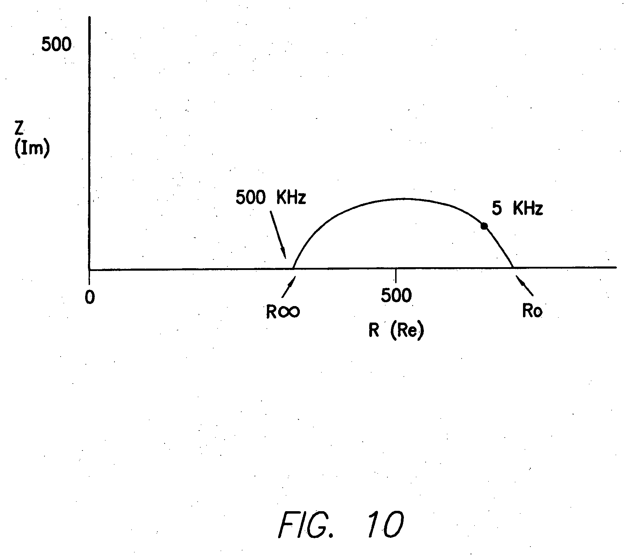

Figure 10 shows a Cole-Cole plot representing the Cole-Cole calculation performed in the process embodiment of Figure 7. The Cole-Cole plot is a useful method for illustrating the behavior of tissue impendance as a function of frequency (See Cole KS, Cole RH, : "Dispersion and absorption in dielectrics," J. Chem. Physics 9: 341-51 (1941)). In the Cole-Cole plot, real component *R* is plotted versus imaginary component X in the complex series impedance (*R* + *jX*) with the frequency as a parameter. The Cole-Cole plot provides a compact, precise layout combining the resistance and reactance plots. Values of ICW and ECW for the patient can be determined using an algorithm equivalent in function to the graphical process which will now be described.

In a Cole-Cole plot, as shown in Figure 10, the resistance is plotted around the X axis and the reactance is plotted on the Y-axis. The plot begins on the right hand side with graphing the resistance and reactance at 5 kHz. The data collected in the sweeps is plotted, and as the frequency is increased, the curve goes up to the left and finally comes down to a zero reactance. The dotted arc beginning at 5 kHz and going to the X-axis is an extrapolation of the data to produce an approximately semicircular graphical representation. The extrapolation of the semicircle to the X axis produces an X-axis intercept that represents R₀, the DC resistance. Because of polarization that would occur if the electrode was at DC, this value must be extrapolated rather than being directly measured. The point at which the resistance intercepts the X axis at the high-frequency side is R_{∞}. In the example of Figure 10 R_{∞} is approximately 500 kHz. R_{∞} typically varies between 200 kHz and 1 MHz depending on the patient.

As noted above with reference to Figure 8, an ECW resistance value is proportional to the resistance R₀ at f=0, which is equal to Rₑ. The resistance R_{∞} at f=∞ is equal to the parallel combination of Rᵢ and Rₑ. By performing the standard electrical engineering calculation for parallel resistance using the collected data, the value of Rᵢ can be calculated, thus giving the ICW estimate. The estimates of ECW and ICW provide an indication of the level of body impedance that can be monitored over time to identify undesirable trends.

## Claims

1. A system for monitoring a patient using an implantable cardiac device (ICD) (10), comprising: monitoring means (108,109) for sensing a position of the patient and making a determination of when the patient assumes a predetermined repose position for at least a predetermined period of time; control means (60) for initiating a plurality of measurements of thoracic impedance after making the determination that the patient is in the predetermined repose position; impedance measuring means (112) for obtaining the plurality of measurements of thoracic impedance by transmitting a signal on at least one ICD lead (302,304) and sensing a received signal at a point separated from the ICD lead; and processing means (81) for calculating, using the measurements, at least one quantity related to thoracic impedance.

2. A system as claimed in Claim 1, **characterised by** diagnostic indication means for providing information useful in diagnosing the patient, using the quantity related to thoracic impedance.

3. A system as claimed in Claim 1 or Claim 2, **characterised in that** the impedance measuring means (112) further comprises frequency varying means for varying the frequency of the signal transmitted on the ICD lead over the plurality of measurements.

4. A system as claimed in Claim 3, **characterised in that** the frequency varying means varies the frequency during the plurality of measurements between a single digit and three digits of magnitude, measured in kilohertz.

5. A system as claimed in Claim 3, **characterised in that** the frequency varying means sequentially varies said frequency in a sweep between approximately 5 kHz and approximately 500 kHz during said plurality of measurements.

6. A system as claimed in Claim 5, **characterised by** averaging means for collecting data from a plurality of the sweeps and averaging the results.

7. A system as claimed in any preceding Claim, **characterised in** the at least one ICD lead comprises a ventricular pacemaker lead ring electrode (34) and/or an atrial pacemaker lead ring electrode (23) and/or a ventricular pacemaker lead ring electrode (34) connected in parallel to an atrial pacemaker lead ring electrode (23).

8. A system as claimed in any preceding Claim, **characterised in that** the said impedance measuring means (112) senses the received signal at an atrial tip pacemaker lead (22) and/or at a ventricular tip pacemaker lead (32) and/or from a parallel connection of an atrial tip pacemaker lead (22) and a ventricular tip (32) pacemaker lead.

9. A system as claimed in any preceding Claim, **characterised by** calculating means for calculating an extracellular water (ECW) resistance value and an intracellular water (ICW) resistance value based on the measurements of thoracic impedance.

10. A system as claimed in Claim 9, **characterised in that** the said ECW and ICW values are determined using a Cole-Cole calculation method.

11. A system as claimed in any preceding Claim, **characterised by** telemetry means (100) for transmitting the quantity used to diagnose the patient to an external device (102) via telemetry.

12. A system as claimed in Claim 11, **characterised by** trend monitoring means for storing (94), over time, repeated measurements of the quantity used to diagnose the patient over time, to provide trend data for the quantity.

## Patentansprüche

1. System zur Überwachung eines Patienten unter Verwendung einer implantierbaren Herzvorrichtung (ICD) (10), aufweisend: Überwachungsmittel (108,109) zum Erfassen einer Position des Patienten und zum Feststellen, wann der Patient eine vorgegebene Ruhestellung während mindestens eines vorgegebenen Zeitraums einnimmt; Steuermittel (60) zum Auslösen mehrerer Messungen der thorakalen Impedanz nach der Feststellung, dass der Patient sich in der vorgegebenen Ruhestellung befindet; Impedanzmessmittel (112) zum Erhalten der mehreren Messungen der thorakalen Impedanz durch das Übertragen eines Signals über mindestens eine ICD-Leitung (302,304) und das Erfassen eines empfangenen Signals an einer von der ICD-Leitung getrennten Stelle; und Verarbeitungsmittel (81) zum Berechnen unter Verwendung der Messungen mindestens einer auf die thorakale Impedanz bezogenen Messgröße.

2. System nach Anspruch 1, **gekennzeichnet durch** Diagnoseanzeigemittel für das Bereitstellen von nützlicher Information beim Diagnostizieren des Patienten unter Verwendung der Messgröße bezüglich der thorakalen Impedanz.

3. System nach Anspruch 1 oder Anspruch 2, **dadurch gekennzeichnet, dass** das Impedanzmessmittel (112) ferner Frequenzveränderungsmittel zum Verändern der Frequenz des auf die ICD-Leitung über die mehreren Messungen übertragenen Signals aufweist.

4. System nach Anspruch 3, **dadurch gekennzeichnet, dass** das Frequenzveränderungsmittel die Frequenz während der mehreren Messungen zwischen Größenordnungen von einer einzelnen Stelle und von drei Stellen, in Kilohertz gemessen, verändert.

5. System nach Anspruch 3, **dadurch gekennzeichnet, dass** das Frequenzveränderungsmittel die Frequenz sequenziell in einem Sweep zwischen etwa 5 kHz und etwa 500 kHz während der mehreren Messungen verändert.

6. System nach Anspruch 5, **gekennzeichnet durch** Mittelungsmittel zum Erfassen von Daten aus mehreren Sweeps und zum Mitteln der Ergebnisse.

7. System nach einem der vorhergehenden Ansprüche, **dadurch gekennzeichnet, dass** die mindestens eine ICD-Leitung eine Kammerschrittmacherleitung-Ringelektrode (34) und/oder eine Vorhofschrittmacherleitung-Ringelektrode (23) und/oder eine Kammerschrittmacherleitung-Ringelektrode (34) aufweist, welche parallel mit einer Vorhofschrittmacherleitung-Ringelektrode (23) verbunden ist.

8. System nach einem der vorhergehenden Ansprüche, **dadurch gekennzeichnet, dass** die Impedanzmessmittel (112) das empfangene Signal an einer Vorhofspitzen-Schrittmacherleitung (22) und/oder an einer Kammerspitzen-Schrittmacherleitung (32) und/oder aus einer Parallelschaltung einer Vorhofspitzen-Schrittmacherleitung (22) und einer Kammerspitzen-Schrittmacherleitung (32) erfasst.

9. System nach einem der vorhergehenden Ansprüche, **gekennzeichnet durch** Berechnungsmittel zum Berechnen eines Widerstandswerts des extrazellulären Wassers (ECW) und eines Widerstandswerts des intrazellulären Wassers (ICW) auf der Grundlage der Messungen der thorakalen Impedanz.

10. System nach Anspruch 9, **dadurch gekennzeichnet, dass** die ECW- und ICW-Werte unter Verwendung eines Cole-Cole-Berechnungsverfahrens bestimmt werden.

11. System nach einem der vorhergehenden Ansprüche, **gekennzeichnet durch** Telemetriemittel (100) zum Übertragen der Messgröße, welche zum Diagnostizieren des Patienten verwendet wird, an eine äußere Vorrichtung (102) **durch** Telemetrie.

12. System nach Anspruch 11, **gekennzeichnet durch** Trendüberwachungsmittel zum Speichern (94) von mehrmaligen Messungen der Messgröße im Zeitablauf, welche zum Diagnostizieren des Patienten im Zeitablauf verwendet wird, um Trenddaten für die Messgröße bereitzustellen.

## Revendications

1. Système pour surveiller un patient à l'aide d'un dispositif cardiaque implantable (DCI) (10), comprenant : des moyens de surveillance (108, 109) pour détecter une position du patient et réaliser une détermination du moment auquel le patient prend une position de repos prédéterminée pendant au moins une période de temps prédéterminée ; des moyens de commande (60) pour initier une pluralité de mesures d'impédance thoracique après avoir réalisé la détermination que le patient est dans la position de repos prédéterminée ; des moyens de mesure d'impédance (112) pour obtenir la pluralité de mesures d'impédance thoracique par transmission d'un signal sur au moins un conducteur de DCI (302, 304) et détection d'un signal reçu à un point séparé du conducteur de DCI ; et des moyens de traitement (81) pour calculer, à l'aide des mesures, au moins une quantité associée à l'impédance thoracique.

2. Système selon la revendication 1, **caractérisé par** des moyens d'indication de diagnostic pour fournir des informations utiles dans le diagnostic du patient, utilisant la quantité associée à l'impédance thoracique.

3. Système selon la revendication 1 ou la revendication 2, **caractérisé par le fait que** les moyens de mesure d'impédance (112) comprennent en outre des moyens de variation de fréquence pour faire varier la fréquence du signal transmis sur le conducteur de DCI sur la pluralité de mesures.

4. Système selon la revendication 3, **caractérisé par le fait que** les moyens de variation de fréquence font varier la fréquence pendant la pluralité de mesures entre un seul chiffre et trois chiffres de grandeur, mesurée en kilohertz.

5. Système selon la revendication 3, **caractérisé par le fait que** les moyens de variation de fréquence font varier séquentiellement ladite fréquence dans un balayage entre approximativement 5 kHz et approximativement 500 kHz pendant ladite pluralité de mesures.

6. Système selon la revendication 5, **caractérisé par** des moyens d'établissement de moyenne pour collecter des données à partir d'une pluralité des balayages et établir une moyenne des résultats.

7. Système selon l'une quelconque des revendications précédentes, **caractérisé par le fait que** le au moins un conducteur de DCI comprend une électrode annulaire de conducteur de stimulateur cardiaque ventriculaire (34) et/ou une électrode annulaire de conducteur de stimulateur cardiaque auriculaire (23) et/ou une électrode annulaire de conducteur de stimulateur cardiaque ventriculaire (34) montée en parallèle à une électrode annulaire de conducteur de stimulateur cardiaque auriculaire (23) .

8. Système selon l'une quelconque des revendications précédentes, **caractérisé par le fait que** lesdits moyens de mesure d'impédance (112) détectent le signal reçu à un conducteur de stimulateur cardiaque de pointe auriculaire (22) et/ou à un conducteur de stimulateur cardiaque de pointe ventriculaire (32) et/ou à partir d'un montage en parallèle d'un conducteur de stimulateur cardiaque de pointe auriculaire (22) et d'un conducteur de stimulateur cardiaque de pointe ventriculaire (32) .

9. Système selon l'une quelconque des revendications précédentes, **caractérisé par** des moyens de calcul pour calculer une valeur de résistance d'eau extracellulaire (ECW) et une valeur de résistance d'eau intracellulaire (ICW) sur la base des mesures d'impédance thoracique.

10. Système selon la revendication 9, **caractérisé par le fait que** lesdites valeurs ECW et ICW sont déterminées à l'aide d'une méthode de calcul de Cole-Cole.

11. Système selon l'une quelconque des revendications précédentes, **caractérisé par** des moyens de télémétrie (100) pour transmettre la quantité utilisée pour diagnostiquer le patient à un dispositif externe (102) par télémétrie.

12. Système selon la revendication 11, **caractérisé par** des moyens de surveillance de tendance pour stocker (94), au fil du temps, des mesures répétées de la quantité utilisée pour diagnostiquer le patient au cours du temps, pour fournir des données de tendance pour la quantité.
